Europäisches Patentamt

European Patent Office

Office européen des brevets

(19)

(11) Publication number : **0 474 374 A1**

## EUROPEAN PATENT APPLICATION

(12)

(21) Application number : **91307335.9**

(22) Date of filing : **09.08.91**

(51) Int. Cl.⁵ : **A61K 31/565,** A61K 31/57

(30) Priority : **10.08.90 US 565763**

(43) Date of publication of application :
**11.03.92 Bulletin 92/11**

(84) Designated Contracting States :
**AT BE CH DE DK ES FR GB GR IT LI LU NL SE**

(71) Applicant : **ORTHO PHARMACEUTICAL
CORPORATION
U.S. Route 202 P.O. Box 300
Raritan New Jersey 08869-0602 (US)**

(72) Inventor : **Demarest, Keith T.
28 Locktown School Road
Flemington, NJ 08822 (US)**
Inventor : **Baylink, David J.
1534 Fern Avenue West
Redlands, California 92373 (US)**

(74) Representative : **Fisher, Adrian John et al
CARPMAELS & RANSFORD 43 Bloomsbury
Square
London WC1A 2RA (GB)**

(54) Method for treatment of bone wasting diseases using a progestin.

(57)    A therapeutic method for the treatment of decreases in bone mass (osteopenias) by the administration of selected progestins is described.

EP 0 474 374 A1

## BACKGROUND OF THE INVENTION

This invention relates to a new therapeutic method for the treatment of decreases in bone mass (osteopenias) which lead to an increase in the risk of bone fracture and back/joint pain associated with these conditions. Osteopenia is a general term used to describe any bone-wasting disease, such as, for example, periodontal disease and osteoarthritis, in which the rate of bone resorption is greater than the rate of bone formation. In particular, the invention relates to a method for treating post-menopausal osteoporosis by the administration of sub-progestational doses of norethindrone, norethindrone acetate, chlormadionone acetate, megestrol acetate, norethynodrel, medroxyprogesterone acetate, and progesterone.

Osteoporosis is a bone-wasting disease in which there is an imbalance or uncoupling between the rate of bone formation and resorption resulting in a decrease in total bone mass. As a result of this decrease in bone mass the human skeleton becomes weakened and unable to bear the normal weight-bearing stresses. The effects of osteoporosis are generally seen in the weight-bearing part of the skeleton, especially the spine and hips, which can fracture in the absence of trauma. Osteoporosis affects about 24 million people in the United States and about 200 million people worldwide. Osteoporosis is believed to be the cause of 2.5 million bone fractures a year in elderly women. The American Medical Association estimates that 25% of white women will suffer fractures of their hip or spine during their lifetime as a result of osteoporosis.

The current therapies for postmenopausal osteoporosis consist of treatments which are, for the most part, preventative; estrogen replacement, bisphosphonates, vitamin D metabolites and calcium supplements all act to inhibit bone resorption associated with the onset of menopause. Estrogen replacement in these patients is quite effective in reducing further loss of bone mass but it does not induce an increase in bone mass which is needed to reduce fracture risk and pain. These treatments have little utility in the treatment of those patients with existing osteoporosis-induced loss of bone mass which have a high fracture risk and back/joint pain. Post-menopausal women with vertebral bone mass of less than 100 mg/cc would be considered below the "fracture threshold" and would be candidates for treatment with an agent which would increase bone mass and thereby restore lost bone.

## SUMMARY OF THE INVENTION

The present invention relates to agents which will increase bone mass and thus reduce or eliminate the risk of bone fracture. The therapeutic need for an agent of this type is clearly present, especially when one considers the poor patient compliance associated with estrogen replacement therapies.

It is well established that estrogens prevent bone loss and alleviate menopausal symptoms in postmenopausal women. [Horseman et al., Br. Med. J., 789-92 (1977)] Estrogens prevent postmenopausal bone loss primarily by reducing bone resorption with a subsequent reduction in bone formation; [Riggs et al., J. Clin. Invest., 51, 1649-63 (1972] thus bone mass is kept constant. The combination of a progestin with estrogen replacement therapy has been suggested in order to reduce the risk of endometrial carcinoma induced by unopposed estrogen, however progestins alone have been shown to significantly decrease the bone loss associated with menopause. Progesterone and medroxyprogesterone acetate have both been shown to reduce bone loss in postmenopausal patients, as evidenced by a decrease in urine calcium and hydroxyproline and by prevention of the decrease in bone mineral content. [Lindsay et al., Clin. Sci., 54, 193-95 (1978] [Christiansen et al., The Lancet, 459-61 (1985] Several other synthetic progestins are effective in preventing bone mineral loss at relatively high doses, e.g. ethynodiol diacetate, lynestrenol, norethindrone and norethindrone acetate. Thus progestins alone or in combination with an estrogen have been described as useful only in preventing the bone loss associated with postmenopausal osteoporosis. U.K. Patent Application No. GB 2216420A describes the use of a progestin unsubstituted in the 19 position for the treatment of osteoporosis on a non-cyclical basis with or without an estrogen. This patent application claims all progestins unsubstituted in the 19 position, preferably in combination with an estrogen, and at doses which exert profound progestational activity. The techniques employed in the application, although they do demonstrate an increase in bone mass in norethindrone/estrogen treated subjects compared to placebo alone treated subjects, do not distinguish the ability of the claimed progestins to inhibit bone resorption from their ability to stimulate actual bone formation. In contrast to these previous disclosures, the present invention demonstrates unexpectedly that a) progestins directly stimulate bone forming cells, osteoblasts, to increase bone formation, b) only certain progestins (norethindrone, norethindrone acetate, chlormadionone acetate, megestrol acetate, norethynodrel, medroxyprogesterone acetate, and progesterone) have this bone forming activity and c) these select progestins can be administered alone in the absence of exogenous estrogen, in a dose range well below that which exhibits progestational activity, to increase bone formation and thus replenish the bone loss associated with menopause. For purposes of the present invention, a progestational dose is a clinically useful dose at which progestins are conventionally used in contraception and hormonal replacement therapy, either alone or in combination with an estrogen. Thus the invention relates to those effects of norethindrone and other select progestins and progestin like compounds that have unique bone forming properties at sub-progestational doses.

The progestins employed in this invention uniquely increase bone formation and thus replenish the bone loss associated with menopause. As a result of this invention it has been demonstrated that these select progestins are effective in stimulating the bone forming cells in humans, mice and white-leg horn chickens. According to this invention, norethindrone, norethindrone acetate, chlormadionone acetate, megestrol acetate, norethynodrel, medroxyprogesterone acetate, and progesterone significantly and reproducibly stimulate osteoblast proliferation and differentiation. The ability to stimulate osteoblasts is not possessed by all progestins. Progestins which do not stimulate bone forming cells include levonorgestrel, desogestrel, gestodene, norgestimate, cyproterone acetate, and 19-nortestosterone. In addition, the action of these selected progestins in stimulating osteoblast proliferation and differentiation has no correlation to the relative progestational or androgenic potency of these compounds. Thus, a separation of hormonal (progestation and androgenic activity) from osteoblast stimulating activity exists implying a different mechanism of action. The osteoblast stimulating activity of these select progestins is not due to an androgenic action since the most androgenic progestin tested, levonorgestrel, was inactive. Norethindrone and the other select progestins also stimulate the proliferation of cellular alkaline phosphatase activity in these osteoblast-line cells, a well known biochemical marker of osteoblast differentiation.

The present invention clearly demonstrates that (a) only certain progestins (norethindrone, norethindrone acetate, chlormadionone acetate, megestrol acetate, norethynodrel, medroxyprogesterone acetate, and progesterone) stimulate bone cell proliferation, (b) the mitogenic action is not species-specific, (c) the mitogenic action is not due to an androgenic action, (d) a large dissociation exists between the hormonal (progestational action on uterus and breast tissue) and bone mitogenic dose response curves, suggesting different mechanisms of action, and that (f) norethindrone and other select progestins stimulate bone cell alkaline phosphatase, a marker of bone cell differentiation. These findings demonstrate that norethindrone and other select progestins have a direct stimulatory effect on osteoblast proliferation and differentiation. Thus norethindrone and the other select progestins can be used to increase bone formation in the treatment of osteoporosis and other bone wasting diseases. The methods used to determine the osteoblast-stimulating action of norethindrone and other select progestins and the results obtained are described below.

The progestins of the present invention, i.e. norethindrone, norethindrone acetate, chloramidionone acetate, megestrol acetate, norethynodrel, medroryprogesterone acetate and progesterone, have been shown to stimulate the bone forming cells or osteoblasts which in turn results in an increase in bone formation when administered at sub-progestational levels. They can be employed, therefore, to increase bone formation thereby replenishing the bone loss associated with menopause. The progestins of the present invention, therefore, can be used to treat osteoporosis and other bone wasting disease-induced decreases in bone mass.

The progestins of the present invention may be administered alone or in combination with an estrogen. Suitable estrogens which may be employed include estropipate, estradiol, estrone and estriol. The presence of the estrogen serves to prevent menopausal related symptoms (hot flash, vaginal atrophy, etc.) and prevents any further bone resorption, but does not interfere with the progestin-induced increase in bone formation. Pharmaceutical compositions containing a progestin of the present invention as the active ingredient in intimate admixture with a pharmaceutical carrier can be prepared according to conventional pharmaceutical compound techniques. The carrier may take a wide variety of forms depending on the form of preparation desired for administration, e.g., oral, buccal, vaginal, topical or parenteral. The composition may also be administered by means of an aerosol. In preparing the compositions in oral dosage form, any of the usual pharmaceutical media may be employed, such as, for example, water, glycols, oils, alcohols, flavoring agents, preservatives, coloring agents and the like in the case of oral liquid preparations (such as, for example, suspensions, elixirs and solutions); or carriers such as starches, sugars, diluents, granulating agents, lubricants, binders, disintegrating agents and the like in the case of oral solid preparations (such as, for example, powders, capsules and tablets). Because of their ease in administration, tablets and capsules represent the most advantageous oral dosage unit form, in which case solid pharmaceutical carriers are employed. If desired, tablets may be sugar-coated or enteric-coated by standard techniques. For parenterals, the carrier will usually comprise sterile water, though other ingredients, for example, to aid solubility or for preservative purposes, may be included. Conventional sustained release methods may also be employed.

The actual sub-progestational dosage employed will vary according to the particular progestin selected, the mode of administration and the nature and severity of the condition being treated.

The pharmaceutical compositions will generally contain per oral dosage unit, e.g., tablet, capsule, powder, liquid and the like from about 0.01 ug/kg to about 200 ug/kg, and preferably from about 0.1 ug/kg to about 6 ug/kg of the progestin for example, norethindrone. The actual amount of each progestin employed will depend upon the bone forming properties of the progestin selected. The dosage range for other routes of administration, e.g. buccal, trasnsdermal, vaginal and other parenteral routes will depend upon the amount of the progestin required to achieve the same range of blood levels and bone forming activity achieved with the oral doses. It is preferred that the dosage levels of the progestins which are the subject of this invention should be sufficient to achieve bone forming activity equivalent to that achieved by the administration of norethindrone when administered at sub-progestational levels.

The methods used to describe the unique unexpected osteoblast-stimulating action of norethindrone and other select progestins and the results obtained are described below.

## METHODS

### Osteoblast Cell Proliferation

The action of select progestins to stimulate osteoblast proliferation can be measured in culture by estimating the rate of DNA synthesis by the rate of 3H-thymidine incorporation into DNA. Only cells undergoing mitosis will synthesize new DNA and thus only these cells will incorporate the radiolabelled DNA specific nucleotide, thymidine. The stimulation of the proliferation and differentiation of bone-forming cells, osteoblasts, is a prerequisite for an increase in bone formation and bone mass. The ability of agents to increase osteoblast proliferation and differentiation can be predicted by their action on cultured osteoblast-line cells in vitro. In this test mouse (MC3T3-EI), and human (TE-85) osteoblast-line cells were cultured in vitro and the effect of various progestins was tested on osteoblast cell proliferation. Osteoblast cell proliferation was determined by measuring the rate of DNA synthesis, which was quantified by measuring the rate of $^3$H-thymidine incorporation into DNA. The specific details of the cell culture condition are given in the examples.

MC-3T3-EI (cloned by Sudo and Coworkers, Koriyama Japan) and TE85 (American Type Tissue Culture Collection #CRL 1543, Rockville, MD) osteoblast-line cells were harvested from large culture flasks (175 cm$^2$) where they were allowed to grow to near confluency using Trypsin. The cells were plated into 96 well culture plates, 1600 cells in 100 ul per well in DMEM (Dulbecos Modified Eagle's Medium) with 25 mM Hepes buffer, L-glutamine (584 mg/l); D-glucose (4.5 gm/l) supplemented with fetal bovine sera (10%); penicillin (100 units/ml) and streptomycin (100 mcg/ml); sodium pyruvate (10 uM final concentration). The cells were allowed to plate overnight in DMEM containing 10% fetal bovine sera at 37°C in an atmosphere of 5% $CO_2$/95% air. Following their placement into 96 well culture plates all the osteoblast-line cells, either the MC3T3-EI or the TE-85 cell lines, were allowed an additional 24 hours preincubation period in media containing only 0.1% fetal bovine sera. The next day the test compounds were added and screened at concentrations ranging from $10^{-14}$ to $10^{-6}$ M depending on the particular study. Twenty hours later a 20 ul aliquot of media containing 0.4 uCi of $^3$H-thymidine was added to each culture well. The cells were then incubated for an additional 4 hours. The incubation was terminated by aspirating the media and washing with HBSS (Hank's Balanced Salt Solution). The cells were then treated with 100 ul of Trypsin-EDTA (Ethylene Diamine Tetra Acetic Acid) (0.5% trypsin and 5.3 mM EDTA) for 30 minutes at room temperature. The cells were then aspirated onto a glass fiber filter and washed with water. The radioactivity on the filters was quantified by liquid scintillation spectroscopy. The rate of $^3$H-thymidine incorporation into DNA is then utilized as an index of cell proliferation.

### Breast Carcinoma Cell Proliferation

T47D breast (Human) carcinoma-line cells (American Type Tissue Culture Collection #HTB 133, Rockville, MD) were harvested using Trypsin from large (175 cm$^2$) culture flasks where they were allowed to grow to near confluency. The cells were plated into 96 well culture plates, 1600 cells in 100 ul per well in DMEM with 25 mM Hepes buffer, L-glutamine (584 mg/l); D-glucose (4.5 gm/l) supplemented with 10% fetal bovine sera; penicillin (100 units/ml) and streptomycin (100 mcg/ml) ; sodium pyruvate (10 uM final concentration). The cells were allowed to plate overnight in DMEM containing 10% fetal bovine sera at 37°C in an atmosphere of 5% $CO_2$/95% air. Following their placement into 96 well culture plates, the cells were incubated for an additional 24 hour preincubation period in media containing only 0.1% fetal bovine sera. The next day the test compounds were added and screened at concentrations ranging from $10^{-14}$ to $10^{-6}$ M depending on the particular study. Twenty hours later a 20 ul aliquot of media containing 0.4 uCi of $^3$H-thymidine was added to each culture well. The cells were then incubated for an additional 4 hours. The incubation was terminated by aspirating the media and washing with HBSS. The cells were then treated with 100 ul of Trypsin-EDTA (0.5% trypsin and 5.3 mM EDTA) for 30 minutes at room temperature, aspirated onto a glass fiber filter and washed with water. The radioactivity on the filters was quantified by liquid scintillation spectroscopy. The rate of $^3$H-thymidine incorporation into DNA is then utilized as an index of cell proliferation.

## Progesterone Receptor Binding

The mechanism of progesterone action has been studied extensively and it is well established that progesterone action is initiated by binding to specific receptors. Synthetic progestogens, like progesterone, exert their action by binding to progestogen specific receptors. The ability of synthetic progestins to bind to the progestin receptor is a prerequisite for its biological activity and is often used as a predictive model of progestational activity. A competitive progestin receptor assay was carried out to demonstrate the relative progestational activity of norethindrone and selected progestins. Progestin receptors were isolated from rabbit uteri which possess soluble cytoplasmic proteins demonstrating a high affinity for progestational agents. Immature rabbits which have been primed with exogenous estrogen to induce proliferation of progestin receptors were used as the tissue source. The cytosolic receptors were incubated in the presence of a specific radiolabelled synthetic progestin, R5020. [Promegestone, New England Nuclear DuPont (#NET-555) catalog number from New England Nuclear DuPont.] After incubation at 4°C the bound R5020 is separated from the free R5020 by absorption to dextran-coated charcoal and the free radiolabelled R5020 was quantified by liquid scintillation spectroscopy. The ability of the test compound to bind to the progestin receptor was determined by its relative ability to displace or compete for the binding of $^3$H-R5020.

Cytosol progestin receptor was prepared from immature female New Zealand White rabbits (0.8-1.0 kg) primed with 17$\beta$ estradiol (5 ug in sesame oil; s.c.) daily for 5 days, the uteri were removed, washed, and homogenized with ice cold Tris-EDTA buffer pH 7.4. The homogenate was centrifuged at 105,000 g for 60 minutes at 4°C and the supernatant was used as the progestin cytosolic receptor preparation. A competitive binding assay was performed by incubating aliquots of the cytosol preparation in the presence of 0.4 nM $^3$H-R5020 and increasing concentrations of unlabelled test progestin for 4 hours at 4°C. At the end of the incubation period, the $^3$H-R5020 bound to the receptor was separated from the unbound $^3$H-R5020 by adsorption on Dextran-coated charcoal. The amount of isotope bound to the receptor was then determined by liquid scintillation spectrometry. The percent inhibition of total $^3$H-R5020 binding was calculated for each concentration of test progestin and an IC$_{50}$ was determined as a measure of binding affinity for the progestin receptor.

## Androgen Receptor Binding

The action of some progestins to bind to an androgen receptor has been used as a predictive model of undesired androgenic action (acne, hirsutism etc). Since several studies have suggested that androgens themselves possess osteoblast stimulating properties it was important to consider whether the osteoblast stimulating properties of norethindrone and related progestins is the result of androgenic properties. A competitive androgen receptor assay was carried out to demonstrate the relative androgenic activity of norethindrone and selected progestins relative to their osteoblast stimulating properties. Androgen receptors were isolated from rat ventral prostates which possess soluble cytoplasmic proteins demonstrating a high affinity for androgens. 5-alpha-dihydrotestosterone (DHT), a naturally occurring testosterone metabolite, has a high binding affinity for these receptors. The relative binding affinity of other steroids, such as progestins, for androgen receptors can be determined by evaluating their ability to displace $^3$H-DHT from these rat ventral prostate receptors in vitro. Cytosolic androgen receptors were prepared from ventral prostate glands isolated from castrated male rats (250-300 g). Ventral prostate glands were homogenized and centrifuged at 105,000 g for 60 min at 4°C and the supernatant was used as the cytosolic androgen receptor preparation. The competitive binding assay was performed by incubating aliquots of cytosol preparation in the presence of 1 nM $^3$H-DHT and increasing concentrations of the unlabelled test progestin in TRIS-EDTA buffer pH 7.4 for 24 hours at 4°C. At the end of the incubation period, the $^3$H-DHT bound to the receptor was separated from unbound $^3$H-DHT by adsorption on dextran-coated charcoal and quantified by liquid scintillation spectrometry. The relative degree of receptor binding of the test progestin was expressed as percent inhibition of total $^3$H-DHT binding.

The following examples describe the invention in greater particularity and are intended to be a way of illustrating but not limiting the invention.

## EXAMPLES

### Example #1 - Progestins on Osteoblast Cell Proliferation

Mouse (MC3T3-El) osteoblast-line cells were cultured in vitro and the effect of various progestins on the ability of the cells to incorporate 3H-thymidine was determined. Osteoblast cell proliferation is determined by measuring the rate of DNA synthesis which was quantified by measuring the rate of $^3$H-thymidine incorporation into DNA. MC-3T3-El osteoblast-line cells were harvested from large culture flasks where they were allowed to grow to near confluency using Trypsin. The cells were plated into 96 well culture plates, 1600 cells in 100 ul per well in DMEM with 25 mM Hepes buffer, L-glutamine 584 mg/l; D-glucose (4.5 gm/l) supplemented with 10% fetal bovine sera; penicillin (100 units/ml) and streptomycin (100 mcg/ml) ; sodium pyruvate (10 uM final concentration). The cells were allowed to plate overnight in DMEM containing 10% fetal bovine sera at 37°C in an atmosphere of 5% $CO_2$/95% air.

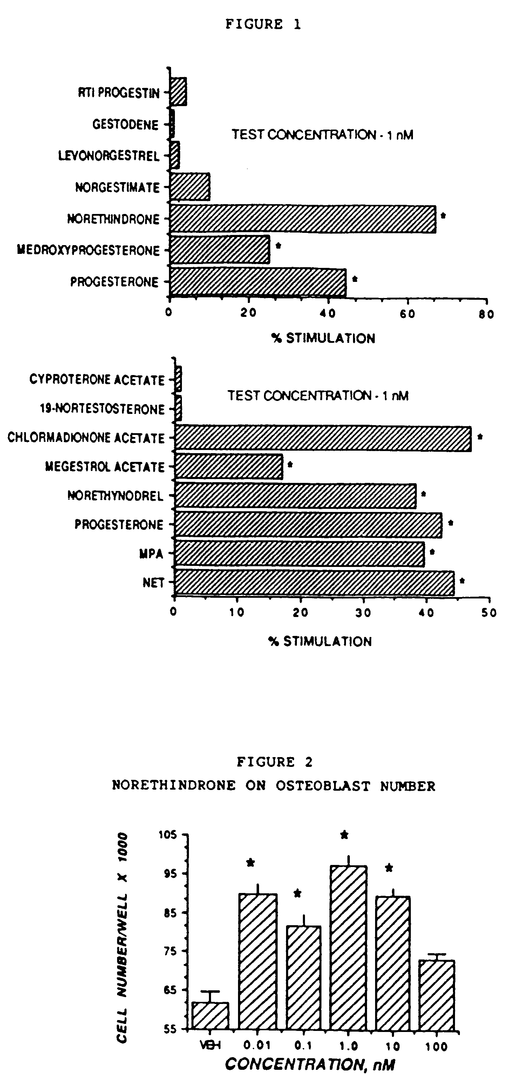

Figure 1. Selected progestins on osteoblast cell proliferation estimated by the rate of $^3$H-thymidine incorporation into DNA. Progestins were incubated with MC3T3-EI cells at concentrations ranging from 100 to 0.001 nM; only the 1 nM concentration is depicted in this figure where the maximal stimulation induced by all progestins was observed. Each bar represents the mean of 8 determinations.

*, indicates values significantly different from vehicle control, p>0.05.

Example #2 - Effect of Norethindrone on Osteoblast Number

A series of studies was undertaken to determine the effect of norethindrone on osteoblast growth as determined by a direct analysis of osteoblast cell number. In this study MC3T3-EI osteoblast-line cells were cultured into 6 well plates at a density of 50,000 cells/well in 10% fetal bovine sera. The following day media was changed to that containing no fetal bovine sera . The next day some of the cells were harvested by trypsin and counted using a Coulter cell counter. This count was considered as Day 0. At the same time other cells were treated with 0.01 to 100 nM of norethindrone and allowed to grow for 5 days with a media change after 48 hours. After 5 days all of the cells were harvested and counted using a Coulter cell counter. Norethindrone induced an increase in osteoblast number confirming the previous studies utilizing 3H-thymidine incorporation as an index of osteoblast proliferation.

Figure 2. Effect of norethindrone on osteoblast cell number estimated by Coulter counting. Norethindrone was incubated for 5 days with MC3T3-EI cells at concentrations ranging from 0.01 to 100 nM. Each striped bar represents the mean and the vertical line I S.E.M. of 6 determinations.

* indicates values significantly different from vehicle control, p>0.05.

Example #3 - Norethindrone on Osteoblast Cell Proliferation and Alkaline Phosphatase Secretion

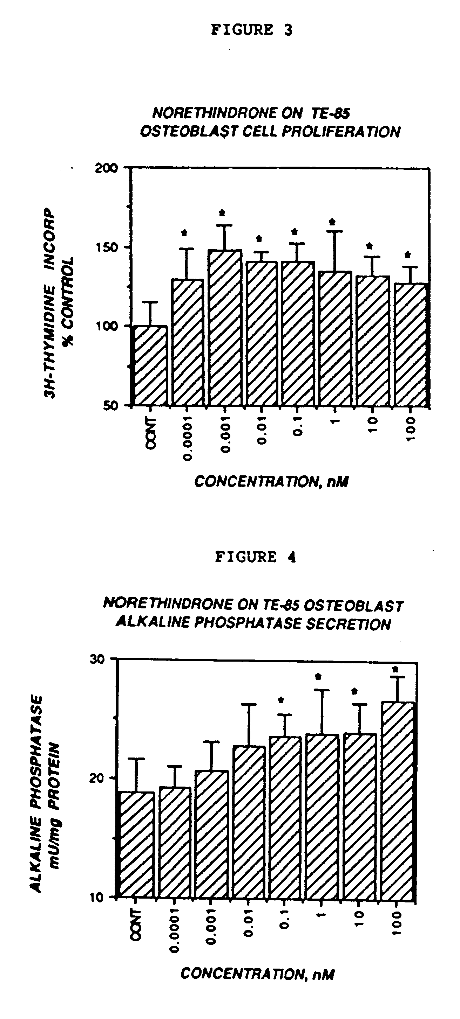

A study was undertaken to determine the effect of norethindrone on osteoblast growth and differentiation. Osteoblast growth was determined by me: ring the rate of 3H-thymidine incorporation into DNA as in Example #1. Osteoblast differention was estimated by measuring the secretion of skeletal alkaline phosphatase into the media. Skeletal alkaline phosphatase is considered a reliable marker of osteoblast differention and activity. TE-85 human osteoblast-line cells were cultured as described previously in Example #1. The osteoblast line cells were treated with either vehicle or the acetate salt of norethindrone at doses ranging from 0.0001 to 100 nM. 3H-Thymidine incorporation into the osteoblast cell DNA and alkaline phosphatase secreted into the media was measured simultaneously (Figure 3 and 4). Norethindrone increased both 3H-thymidine incorporation and secretion of alkaline phosphatase by the TE-85 human osteoblast-line cells. The results confirm that norethindrone increases the differentiation, as well as proliferation of human osteoblast line cells.

Figure 3. Norethindrone acetate on osteoblast cell proliferation estimated by the rate of $^3$H-thymidine incorporation into DNA. Each striped bar represents the mean and the open bar I S.E.M. of 6 determinations. * p>0.05.

Figure 4. Norethindrone acetate on alkaline phosphatase activity in osteoblast-line cells. Each striped bar represents the mean and the open bar I S.E.M. of 6 determinations. * p>0.05.

Example #4 - Comparison of Osteoblast Proliferative Action to Progestational and Androgenic Activities

The ability of norethindrone and selected progestins to stimulate osteoblast proliferation does not correlate with the ability of these compounds to stimulate "classical" progestin or androgen properties in either uterus or prostate tissue respectively, both of which are classical target sites for progestins and androgens. The ability of progestins to bind to either uterine progestin or prostate androgen receptors was compared to their ability to stimulate osteoblast cell proliferation.

Synthetic progestogens exert their action, like progesterone, by binding to progestogen specific receptors. The ability of synthetic progestins to bind to the progestin receptor is a prerequisite for its biological activity and is often used as a predictive model of progestational activity. à competitive progestin receptor assay was carried out to demonstrate the relative progestational activity of norethindrone and selected progestins. Progestin receptors were isolated from rabbit uteri which possess soluble cytoplasmic proteins demonstrating a high affinity for progestational agents. Immature rabbits which have been primed with exogenous estrogen to induce proliferation of progestin receptors were used as the tissue source. The cytosolic receptors were incubated in the presence of a specific radiolabelled synthetic progestin, R5020. After incubation at 4°C the bound R5020 is separated from the free R5020 by absorption on dextran-coated charcoal and the free radiolabelled R5020 was quantified by liquid scintillation spectroscopy. The ability of test compounds to bind to the progestin receptor was determined by their relative ability to displace or compete for the binding of $^3$H-R5020. Cytosol progestin receptor was prepared from immature female New Zealand White rabbits (0.8-1.0 kg) primed with 17 beta-estradiol (5 ug in sesame oil; s.c.) daily for 5 days. The uteri were removed, washed, and homogenized with ice cold Tris-EDTA buffer pH 7.4. The homogenate was centrifuged at 105,000 g for 60 minutes at 4°C and the supernatant was used as the progestin cytosolic receptor preparation. A competitive binding assay was performed by incubating aliquots of the cytosol preparation in the presence of 0.4 nM $^3$H-R5020 and increasing concentrations of unlabelled test progestin for 4 hours at 4°C. At the end of the incubation period, the $^3$H-R5020 bound to the receptor was separated from the unbound $^3$H-R5020 by adsorption on Dextran-coated charcoal. The amount of isotope bound to the receptor was then determined by liquid scintillation spectrometry. The percent inhibition of total $^3$H-R5020 binding was calculated for each concentration of test progestin and a $IC_{50}$ as a measure of binding affinity for the progestin receptor.

The action of some progestins to bind to androgen receptor has been used as a predictive model of a progestin undesired androgenic action (acne, hirsutism etc). A competitive androgen receptor assay was carried out to demonstrate the relative androgenic activity of norethindrone and selected progestins relative to their osteoblast stimulating properties. Androgen receptors were isolated from rat ventral prostates which possess soluble cytoplasmic proteins demonstrating a high affinity for androgens. 5-alpha-dihydrotestosterone (DHT), a naturally occurring testosterone metabolite, has a high binding affinity for these receptors. The relative binding affinity of other steroids, such as progestins, for androgen receptors is determined by evaluating their ability to displace $^3$H-DHT from these rat ventral prostate receptors in vitro. Cytosolic androgen receptors were prepared from ventral prostate glands isolated from castrate male rats (250-300 g). Ventral prostate glands were homogenized and centrifuged at 105,000 g for 60 min at 4°C and the supernatant used as the cytosolic androgen receptor preparation. The competitive binding assay was performed by incubating aliquots of cytosol preparation in the presence of 1 nM $^3$H-DHT and increasing concentrations of the unlabelled test progestin in TRIS-EDTA buffer pH 7.4 for 24 hours at 4°C. At the end of the incubation period, the $^3$H-DHT bound to the receptor was separated from unbound by $^3$H-DHT adsorption on dextran-coated charcoal and quantified by liquid scintillation spectrometry. The relative degree of receptor binding of the test progestin was expressed as percent inhibition of total $^3$H-DHT binding.

Table I is a summary of the results of studies comparing the ability of progestins to bind to either uterine progestin or prostate androgen receptors to their ability to stimulate osteoblast cell proliferation. Those compounds which exhibit the highest affinity toward progestin receptors do not necessarily increase osteoblast proliferation. For example, levonorgestrel is one of the most potent progestins with relatively high affinity for both progestin and androgen receptors but it is devoid of any osteoblast proliferative effects. Norethindrone on the other hand is also a potent progestin which does possess osteoblast proliferative action. The action of norethindrone and the other active progestins to stimulate osteoblast proliferation can not be predicted on the basis of known progestational and/or androgenic potencies.

## TABLE I

### RELATIVE BINDING AFFINITY FOR RABBIT UTERINE PROGESTIN AND RAT PROSTATIC ANDROGEN RECEPTORS

| COMPOUND | OSTEOBLAST STIMULATION | PROGESTIN BINDING IC50, nM | ANDROGEN BINDING IC50, nM |
|---|---|---|---|
| PROGESTERONE | ACTIVE | 4.33+1.1 | 401+41 |
| NORETHINDRONE | ACTIVE | 0.68+0.21 | 45+11 |
| LEVONORGESTREL | NOT ACTIVE | 0.80+0.14 | 13+6 |
| NORGESTIMATE | NOT ACTIVE | 3.48+0.47 | 764+45 |
| GESTODENE | NOT ACTIVE | 0.47+0.11 | 13+2 |
| 3-KETO-DESOGESTREL | NOT ACTIVE | 0.83+0.11 | 77+12 |
| DIHYDROTESTOSTERONE | NOT ACTIVE | 127.15+27.0 | 2+0.2 |

Osteoblast stimulating activity refers to the ability of test compounds to stimulate $^3$H-thymidine incorporation in MC3T3-El osteoblast-line cells (Active ≥ 20% stimulation over control). Relative progestin receptor binding activity was analyzed in competitive binding assays utilizing 3H-R-5020 binding for rabbit uterine progestin cytosolic receptors. Relative androgen receptor binding activity was analyzed in competitive binding assays utilizing 3H-DHT binding for rat prostate androgen cytosolic receptors. Each IC50 value represents the mean + I S.E. of 4 determinations performed in duplicate.

Example #5 - Comparison of norethindrone induced cell proliferation in osteoblasts versus breast line cells

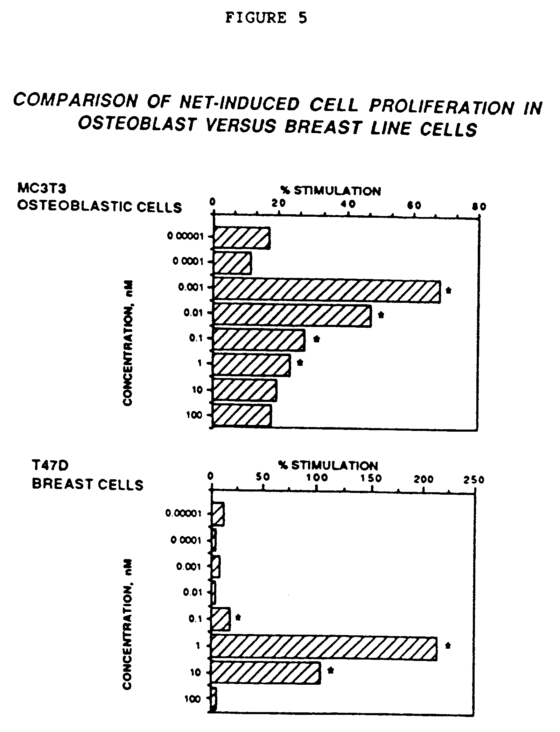

Previous observations indicate that the action of norethindrone to stimulate osteoblast proliferation does not correlate with its ability to stimulate the "classic" progestin receptor. Studies were undertaken to compare the ability of norethindrone to stimulate osteoblast proliferation to proliferation of breast cells, a well known progestin target tissue. In these studies a dose-response relationship for the effect of norethindrone on 3H-thymidine incorporation in osteoblast mouse (MC3T3-El) and breast carcinoma (human) (T47D) cell lines was determined (Figure 5). MC3T3-El cells were cultured as described in Example #1. T47D breast carcinoma-line cells were cultured and tested as follows: the cells were harvested using trypsin from large culture flasks where they were allowed to grow to near confluency The cells were plated into 96 well culture plates, 1600 cells in 100 ul per well in DMEM with 25 mM Hepes buffer, L-glutamine (570 mg/l); D-glucose (4.5 gm/l) supplemented with 10% fetal bovine sera; penicillin (100 units/ml) and streptomycin (100 mcg/ml); sodium pyruvate (10 uM final concentration). The cells were allowed to plate overnight in DMEM containing 10% fetal bovine sera at 37°C in an atmosphere of 5% $CO_2$/95% air. Following their placement into 96 well culture plates the cells were allowed an additional 24 hour preincubation period in media containing only 0.1% fetal bovine sera. The next day the test compounds were added. Norethindrone, dissolved in a final concentration of 0.01% ethanol in culture media, was tested in both the osteoblast and breast cells at concentrations ranging from 0.00001 to 100 nM. Twenty hours later a 20 ul aliquot of media containing 0.4 uCi of $^3$H-thymidine was added to each culture well. The cells were then incubated an additional 4 hours. The incubation was terminated by aspirating the media and washing with HBSS. The cells were then treated with 100 ul of Trypsin-EDTA (0.5% trypsin and 5.3 mM EDTA) for 30 minutes at room temperature. The cells were then aspirated onto a glass fiber filter and washed with water. The radioactivity on the filters was quantified by liquid scintillation spectroscopy. The rate of $^3$H-thymidine incorporation into DNA was then utilized as an index of cell proliferation.

Norethindrone induced a dose-related increase in the proliferation of both osteoblast and breast cell lines. The doses of norethindrone which induce a maximal stimulation of proliferation in breast cells versus osteoblasts is, however, quite different. The maximal effective dose of norethindrone which stimulates breast cell proliferation is in the range of 1-10 nM while the maximally effective dose in osteoblasts is much lower, i.e. 0.01-0.001 nM. This action of progestins to stimulate breast cell proliferation is a well known action of this class of compounds, however, as stated previously, the action to stimulate osteoblast is novel and can not be predicted on the basis of progestional activity. The observation that the maximal stimulatory action on osteoblasts occurs at doses much lower than breast cells is additional evidence that the action on osteoblast is mediated by a different mechanism than the "classic" progestin receptor as in breast cells.

Figure 5. Comparison of norethindrone on cell proliferation in osteoblast-line cells and breast carcinoma line cells estimated by the rate of $^3$H-thymidine incorporation into DNA. Norethindrone was incubated with MC3T3-El and T47D cells at concentrations ranging from 100 to 0.00001 nM. Each striped bar represents the mean and the open bar I S.E.M. of 8 determinations.

* indicates values significantly different from vehicle control, $p > 0.05$.

## Claims

1. The use of a progestin in the manufacture of a medicament for increasing bone formation in the treatment of osteopenias, said progestin being selected from the group consisting of norethindrone, norethindrone acetate, chlormadionone acetate, megestrol acetate, norethynodrel, medroxyprogesterone acetate and progesterone.

2. The use of a progestin in the manufacture of a medicament for increasing bone formation in the treatment of osteoporosis-induced bone loss, said progestin being selected from the group consisting of norethindrone, norethindrone acetate, chlormadionone acetate, megestrol acetate, norethynodrel, medroxyprogesterone acetate and progesterone.

3. The use of a progestin in accordance with claim 1 or claim 2 wherein the progestin is norethindrone.

4. The use of a progestin in accordance with claim 1 or claim 2 wherein the progestin is norethindrone acetate.

5. The use of a progestin in accordance with claim 1 or claim 2 wherein the progestin is norethynodrel.

6. The use of a progestin in accordance with claim 1 or claim 2 wherein the progestin is progesterone.

7. The use of a progestin in accordance with claim 1 or claim 2 wherein the progestin is administered in conjunction with an estrogenic component selected from the group consisting of estropipate, estradiol, estrone and estriol.

8. A progestin for use in the treatment of osteopenias or osteoporosis induced bone loss, said progestin being selected from the group consisting of norethindrone, norethindrone acetate, chlormadionone acetate, megestrol acetate, norethynodrel, medroxyprogesterone acetate and progesterone.

## FIGURE 1

## FIGURE 2
### NORETHINDRONE ON OSTEOBLAST NUMBER

10

FIGURE 3

NORETHINDRONE ON TE-85
OSTEOBLAST CELL PROLIFERATION

FIGURE 4

NORETHINDRONE ON TE-85 OSTEOBLAST
ALKALINE PHOSPHATASE SECRETION

FIGURE 5

## COMPARISON OF NET-INDUCED CELL PROLIFERATION IN OSTEOBLAST VERSUS BREAST LINE CELLS

European Patent Office

**EUROPEAN SEARCH REPORT**

Application Number

EP 91 30 7335

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X,Y | MATURITAS, vol. 8, no. 4, 1986, pages 267-274, Elsevier Science Publishers B.V.; B.J. RIIS et al.: "Post-menopausal bone loss: effects of oestrogens and progestogens. A review" * Whole article * | 1-8 | A 61 K 31/565 A 61 K 31/57 |
| X,Y | AM. J. OBSTET. GYNECOL., vol. 163, no. 2, August 1990, pages 614-618; M.J. JAYO et al.: "Effects on bone of surgical menopause and estrogen therapy with or without progesterone replacement in cynomolgus monkeys" * Whole article * | 1-8 | |
| X,Y | THE LANCET, vol. 2, no. 8459, 12th October 1985, pages 800-801; C. CHRISTIANSEN et al.: "Uncoupling of bone formation and resorption by combined oestrogen and progestagen therapy in postmenopausal osteoporosis" * Whole article * | 1-8 | |
| X,Y | GYNECOL. ENDOCRINOL., vol. 1, no. 2, 1987, pages 169-175; K. THOMSEN et al.: "Bone turnover in postmenopausal women after withdrawal of estrogen/gestagen replacement therapy" * Whole article * | 1-8 | TECHNICAL FIELDS SEARCHED (Int. Cl.5) A 61 K |
| X,Y | EP-A-0 309 263 (JENCAP RESEARCH LTD) * Column 5, lines 28-31; column 8, lines 57-65; column 12, lines 5-35 * | 1-8 | |
| X,Y D | GB-A-2 216 420 (SANDOZ LTD) * Pages 1-4,20-22; claims 1,2,4,5,6,11,15-18 *          -/- | 1-8 | |

The present search report has been drawn up for all claims

| Place of search | Date of completion of the search | Examiner |
|---|---|---|
| THE HAGUE | 21-11-1991 | GAC G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

Page   2

**European Patent Office**

## EUROPEAN SEARCH REPORT

Application Number

EP  91 30 7335

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X,Y | THE JOURNAL OF REPRODUCTIVE MEDICINE, vol. 27, no. 1, 1982, pages 1-28; G.V. UPTON et al.: "The perimenopause physiologic correlates and clinical management" * Pages 13-22 * ----- | 1-8 | |
| | | | TECHNICAL FIELDS SEARCHED (Int. Cl.5) |

The present search report has been drawn up for all claims

| Place of search | Date of completion of the search | Examiner |
|---|---|---|
| THE HAGUE | 21-11-1991 | GAC G. |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P0401)